(19) Europäisches Patentamt
European Patent Office
Office européen des brevets

(11) Veröffentlichungsnummer : **0 117 467**
**B1**

# (12) EUROPÄISCHE PATENTSCHRIFT

(45) Veröffentlichungstag der Patentschrift : **06.05.87**

(21) Anmeldenummer : **84101373.3**

(22) Anmeldetag : **10.02.84**

(51) Int. Cl.⁴ : **C 07 D221/12**, C 07 D493/06, C 07 D401/14, C 07 D401/04, A 61 K 31/435, A 61 K 31/365, A 61 K 31/445

## (54) Polyzyklische Bisamidine, Verfahren zu ihrer Herstellung und ihre Verwendung als Chemotherapeutika.

(30) Priorität : **17.02.83 DE 3305329**

(43) Veröffentlichungstag der Anmeldung : **05.09.84 Patentblatt 84/36**

(45) Bekanntmachung des Hinweises auf die Patenterteilung : **06.05.87 Patentblatt 87/19**

(84) Benannte Vertragsstaaten : **CH DE FR GB IT LI**

(56) Entgegenhaltungen :
**DE-A- 1 645 907**
**DE-B- 1 083 826**
**US-A- 3 953 455**
**CHEMICAL ABSTRACTS, Band 91, Nr. 17, 22. Oktober 1979, Columbus, Ohio, USA G. I. MIGACHEV "Synthesis of 2,7-disubstituted 5,10-dioxo-4,5,9,10-tetrahydro-4-9-dioxapyrenes" Seite 649, Spalte 2, Zusammenfassung Nr. 140 744z**

(73) Patentinhaber : **HOECHST AKTIENGESELLSCHAFT**
**Postfach 80 03 20**
**D-6230 Frankfurt am Main 80 (DE)**

(72) Erfinder : **Bajwa, Balbir Singh, Dr.**
**verstorben (IN)**
Erfinder : **Chatterjee, Dipak Kumar, Dr.**
**G21, Hoechst Officer's Colony**
**Mulund (West) Bombay 400 080 (IN)**
Erfinder : **Ganguli, Bimal Naresh, Dr.**
**"Saurayuth" Central Avenue 173**
**Chembur Bombay 400 071 (IN)**
Erfinder : **Reden, Jürgen, Dr. A 84 Meher Apartments**
**Anstey Road Off Altamount Road**
**Cumballa Hill Bombay 400 026 (IN)**
Erfinder : **de Souza, Noel John, Dr.**
**702 Avanti, Central Avenue**
**Santa Cruz Bombay 400 054 (IN)**

**EP 0 117 467 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Gegenstand der vorliegenden Erfindung sind neue Bisamidin-derivate polyzyklischer Moleküle der Formel I

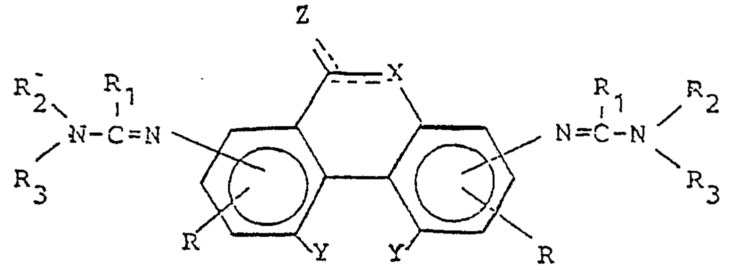

(I)

worin

R Wasserstoff, $C_1$-$C_3$-Alkyl, $C_1$-$C_3$-Alkoxy, Halogen, Nitro oder Amino,

$R_1$ Wasserstoff, eine nicht substituierte oder mit der Amino- oder Di-$C_1$-$C_3$-alkylaminogruppe substituierte $C_1$-$C_6$-Alkylgruppe, oder eine Di-$C_1$-$C_3$-alkylaminogruppe,

$R_2$ und $R_3$ einzeln eine $C_1$-$C_6$-Alkylgruppe oder, zusammen mit dem Stickstoffatom, an das sie gebunden sind, eine heterozyklische Gruppe bedeuten, oder

$R_1$ und $R_2$ zusammen mit dem Kohlenstoffatom und dem Stickstoffatom, an das sie gebunden sind, eine heterozyklische Gruppe bedeuten,

X und Z jeweils Sauerstoff bedeuten oder

X für die —NH-Gruppe und Z für Sauerstoff steht oder

X für Stickstoff und Z für Wasserstoff, Halogen, $C_1$-$C_6$-Alkoxy, Amino, Mono-$C_1$-$C_6$-alkylamino, Di-$C_1$-$C_6$-alkylamino oder eine heterozyklische Gruppe,

Y für Wasserstoff oder, falls X und Z jeweils Sauerstoff bedeuten, für die Brücke

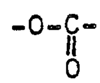

die ein zusätzliches Ringsystem bildet, steht und deren pharmazeutisch verträgliche Salze.

3,8-Bisamin-6-p-aminophenylphenanthridin-Derivate mit trypanociden Wirkung sind bereits aus der DE-B-1 083 826 bekannt.

Als Beispiel für erfindungsgemäß polyzyklische Moleküle ist das 5,10-Dioxo-4,5,9,10-tetrahydro-4,9-dioxapyren, ein tetrazyklisches Molekül der Formel I, worin X und Z Sauerstoff bedeuten, zu nennen.

Gegenstand vorliegender Erfindung sind daher Bisamidin-Derivate der folgenden Formel Ia

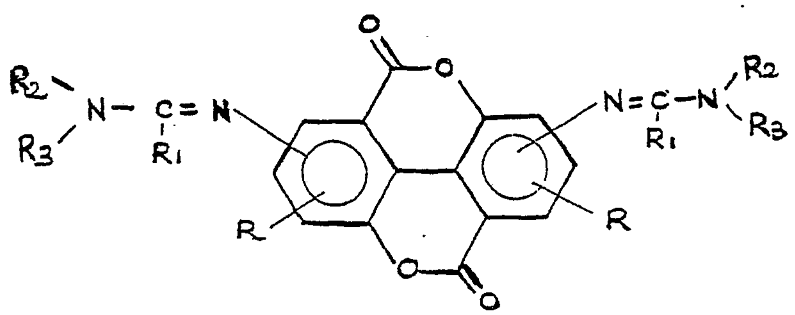

(Ia)

Als Beispiel für erfindungsgemäße polyzyklische Moleküle wäre weiterhin das trizyklische 6 (5H)-Phenanthridinon der Formel I, worin das durch die

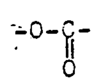

Brücke gebildete Ringsystem fehlt und worin X die NH-Gruppe und Z Sauerstoff bedeuten, zu nennen. Folglich stellen Bisamidinderivate der folgenden Formel Ib einen weiteren erfindungsgemäßen Gegenstand dar :

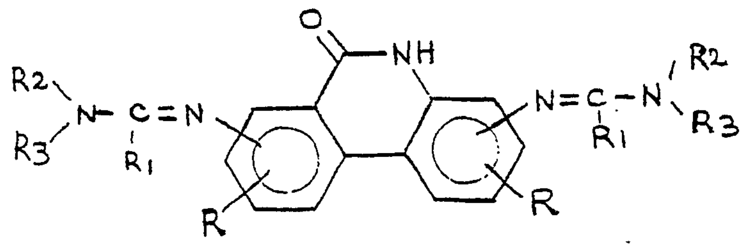

(Ib)

Als dritted Beispiel für polyzyklische erfindungsgemäße Moleküle wäre das trizyklische Phenanthridin der Formel I, worin die die zusätzliche Ringverbindung darstellende

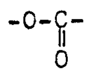

Brücke ebenfalls fehlt, X Stickstoff und Z Wasserstoff, Halogen, $C_1$-$C_6$-Alkoxy, Amino, Mono-$C_1$-$C_6$-alkylamino, Di-$C_1$-$C_6$-alkylamino bedeuten, der folgenden Formel Ic zu nennen :

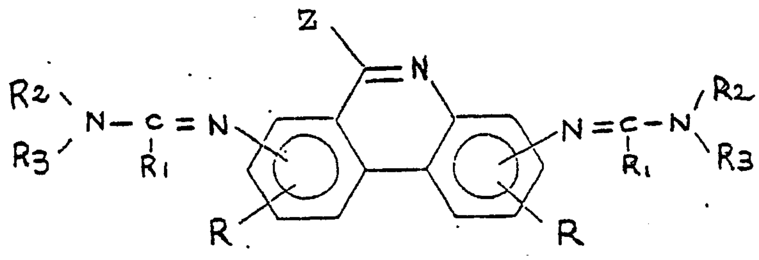

(Ic)

R in den Formeln Ia, Ib und Ic bedeutet Wasserstoff. Falls $R_1$, $R_2$ und $R_3$ für Alkylgruppen stehen, kommen bevorzugt solche mit bis zu 3 Kohlenstoffatomen wie z. B. Methyl, Äthyl, n-Propyl oder Isopropyl in Frage.

Dialkylaminogruppen für $R_1$ sind bevorzugt Dimethylamino oder Diäthylamino.

Falls Z für Alkoxy, Alkylamino oder Dialkylaminogruppen steht, kommen bevorzugt solche mit 1-3 C-Atomen in Frage.

Stickstoffhaltige heterozyklische Gruppen sind bevorzugt Pyrrolidin, Piperidin, Imidazolin oder Pyrimidin, die gegebenenfalls durch eine oder mehrere $C_1$-$C_3$-Alkylgruppen substituiert sind.

Als Beispiele für erfindungsgemäße pharmazeutisch verträgliche Salze der Bisamidinderivate sind anorganische Säureadditionsalze wie Hydrochloride, Hydrojodide, Hydrofluoride, Sulfate oder Phosphate oder organische Säureadditionssalze wie Acetate, Oxalate, Tartrate, Citrate, Maleate, Fumarate oder Methansulfonate, die durch Reaktion mit den entsprechenden Säuren auf übliche Weise erhalten werden, zu nennen.

Als erfindungsgemäße Bisamidinderivate des 5,10-Dioxo-4,5-9,10-tetrahydro-4,9-dioxapyrens der Formel Ia sind solche bevorzugt, worin R Wasserstoff bedeutet und die Amidinoderivate in 2- oder 7-Position stehen, $R_1$ für eine $C_1$-$C_6$-Alkyl- oder Di-$C_1$-$C_6$-alkylaminogruppe steht, $R_2$ und $R_3$ jeweils einzeln eine $C_1$-$C_6$-Alkylgruppe bedeuten oder zusammen mit dem Stickstoffatom, an das sie gebunden sind, einen Heterozyklus bilden, $R_1$ und $R_2$ zusammen mit dem Kohlenstoffatom und dem Stickstoffatom, an das sie gebunden sind, eine heterozyklische Gruppe bedeuten, und pharmazeutisch verträgliche Salze davon.

Als erfindungsgemäße Bisamidinderivate des 5,10-Dioxo-4,5-9,10-tetrahydro-4,9-dioxopyrens der Formel Ia werden insbesondere die folgenden bevorzugt :

1. 2,7-Di-(N',N'-diäthylaminomethylenamino)-5,10-dioxo-4,5,9,10-tetrahydro-4,9-dioxapyren-dihydrochlorid.
2. 2,7-Di-(N',N'-diäthylaminoäthylidenamino)-5,10-dioxo-4,5,9,10-tetrahydro-4,9-dioxapyren.
3. 2,7-Di-(N',N'-diäthylaminoäthylidenamino)-5,10-dioxo-4,5,9,10-tetrahydro-4,9-dioxapyren-dihydrochlorid.
4. 2,7-Di-(N-methylpyrrolidin-2-ylidenamino)-5,10-dioxo-4,5,9,10-tetrahydro-4,9-dioxapyren-dihydrochlorid.
5. 2,7-Di-(N-methylpiperid-2-ylidenamino)-5,10-dioxo-4,5,9,10-tetrahydro-4,9-dioxapyren-dihydrochlorid.
6. 2,7-Di-(piperidinomethylenamino)-5,10-dioxo-4,5,9,10-tetrahydro-4,9-dioxapyren-dihydrochlorid-monohydrat.

In der folgenden Tabelle sind einige neue Bisamidinderivate des 5,10-Dioxo-4,5,9,10-tetrahydro-4,9-dioxapyrens der Formel Ia aufgeführt :

| $R_1$ | $R_2$ | $R_3$ | Salz | Fp. (°) |
|---|---|---|---|---|
| H | $CH_3$ | $CH_3$ | - | 300 |
| H | $CH_3$ | $CH_3$ | 2HCl | 300 |
| H | $C_2H_5$ | $C_2H_5$ | - | 232 - 5 |
| H | $C_2H_5$ | $C_2H_5$ | 2HCl | 282 (Zers.) |
| $CH_3$ | $CH_3$ | $CH_3$ | - | 276 - 8 |
| $CH_3$ | $CH_3$ | $CH_3$ | 2HCl | 298 - 91(Zers.) |
| $CH_3$ | $C_2H_5$ | $C_2H_5$ | - | 220 - 2 |
| $CH_3$ | $C_2H_5$ | $C_2H_5$ | 2HCl | 284 - 6 (Zers.) |
| $CH_3$ | $C_2H_5$ | $C_2H_5$ | $2CH_3SO_3H$ | 286 - 88(Zers.) |
| $CH_3$ | $C_2H_5$ | $C_2H_5$ | $2CH_2COOHCHOHCH_2COOH$ | 198 - 99 |
| $CH_3$ | $C_2H_5$ | $C_2H_5$ | 2HI | 310 (Zers.) |
| $CH_3$ | $C_2H_5$ | $C_2H_5$ | $2H_2SO_4$ | 294 - 96(Zers.) |
| $CH_3$ | $C_3H_7$ | $C_3H_7$ | - | 201 - 3 |
| $CH_3$ | $C_3H_7$ | $C_3H_7$ | 2HCl | 266 - 70(Zers.) |
| $C_2H_5$ | $CH_3$ | $CH_3$ | - | 246 - 8 |
| $C_2H_5$ | $CH_3$ | $CH_3$ | 2HCl | 265 - 7 (Zers.) |
| H | $-(CH_2)_4-$ | | - | 265 - 7 (Zers.) |
| H | $-(CH_2)_4-$ | | $2HCl \cdot H_2O$ | 283 - 5 (Zers.) |
| H | $-(CH_2)_5-$ | | - | 275 (Zers.) |
| H | $-(CH_2)_5-$ | | $2HCl \cdot H_2O$ | 290 - 93(Zers.) |
| $-(CH_2)_3-$ | | $CH_3$ | - | 274 - 75 |
| $-(CH_2)_3-$ | | $CH_3$ | 2HCl | 300 (Zers.) |
| $-(CH_2)_3-$ | | $C_2H_5$ | - | 310 - 11(Zers.) |
| $-(CH_2)_3-$ | | $C_2H_5$ | 2HCl | 300 |
| $-(CH_2)_4-$ | | $CH_3$ | - | 260 - 62 |
| $-(CH_2)_4-$ | | $CH_3$ | 2HCl | 277 - 79 |
| $N(CH_3)_2/CH_3$ | | $CH_3$ | - | 248 - 52 |

Als Bisamidinderivate des 6(5H)-Phenanthridinons der Formel Ib werden solche bevorzugt, worin R für Wasserstoff steht, die Amidinogruppe sich in 3- und 8-Stellung befinden, $R_1$ für Wasserstoff oder eine $C_1$-$C_6$-Alkylgruppe steht, $R_2$ und $R_3$ einzeln jeweils für eine $C_1$-$C_6$-Alkylgruppe stehen oder zusammen mit dem Stickstoffatom, an das sie gebunden sind, einen Heterozyklus bilden oder worin $R_1$ und $R_2$ zusammen mit dem Kohlenstoffatom und dem Stickstoffatom an das sie gebunden sind, einen Heterozyklus bilden, sowie deren pharmazeutisch verträglichen Salze.

Als erfindungsgemäße Bisamidinderivate des 6(5H)-Phenanthridinons der Formel Ib sind die folgenden besonders bevorzugt :

1. 3,8-Di-(N',N'-dimethylaminoäthylidenamino)-6(5H)-phenanthridinon-dihydrochlorid-dihydrat.
2. 3,8-Di-(N',N'-diäthylaminomethylenamino)-6(5H)-phenanthridinon.
3. 3,8-Di-(N',N'-diäthylaminomethylenamino)-6(5H)-phenanthridinon-dihydrochlorid-dihydrat.
4. 3,8-Di-(N'-methylpyrrolidin-2-ylidenamino)-6(5H)-phenanthridinon-dihydrochlorid-dihydrat.
5. 3,8-Di-(piperidinomethylenamino)-(5H)-phenanthridinon-dihydrochlorid.
6. 3,8-Di-(pyrrolidinomethylenamino)-6(5H)-phenanthridinon.

In der folgenden Tabelle sind einige neue Bisamidinderivate des 6(5H)-Phenanthridinons der Formel

Ib aufgeführt :

| $R_1$ | $R_2$ | $R_3$ | Salz | Fp. (°) |
|---|---|---|---|---|
| H | $C_2H_5$ | $C_2H_5$ | - | 240 - 1 (Zers.) |
| H | $C_2H_5$ | $C_2H_5$ | $2HCl \cdot 2H_2O$ | 281 - 3 (Zers.) |
| $CH_3$ | $CH_3$ | $CH_3$ | - | 257 - 9 (Zers.) |
| $CH_3$ | $CH_3$ | $CH_3$ | $2HCl \cdot 2H_2O$ | 268 - 70(Zers.) |
| $CH_3$ | $C_2H_5$ | $C_2H_5$ | - | 220 - 2 |
| $CH_3$ | $C_2H_5$ | $C_2H_5$ | 2HCl | 255 - 7 (Zers.) |
| $C_2H_5$ | $CH_3$ | $CH_3$ | - | 272 - 4 |
| $C_2H_5$ | $CH_3$ | $CH_3$ | $2HCl \cdot 2H_2O$ | 223 - 5 |
| H | $-(CH_2)_4-$ | | - | 280 (Zers.) |
| H | $-(CH_2)_4-$ | | 2HCl | 300 (Zers.) |
| H | $-(CH_2)_5-$ | | - | 152 - 3 |
| H | $-(CH_2)_5-$ | | 2HCl | 255 - 7 (Zers.) |
| $-(CH_2)_3-$ | | $CH_3$ | - | 259 - 61(Zers.) |
| $-(CH_2)_3-$ | | $CH_3$ | $2HCl \cdot 2H_2O$ | 255 (Zers.) |
| $-(CH_2)_3-$ | | $C_2H_5$ | - | 273 - 5 (Zers.) |
| $-(CH_2)_3-$ | | $C_2H_5$ | 2HCl | 290 (Zers.) |
| $-(CH_2)_4-$ | | $CH_3$ | - | 263 - 5 (Zers.) |
| $-(CH_2)_4-$ | | $CH_3$ | $2HCl \cdot 2H_2O$ | 250 - 2 (Zers.) |

Als Bisamidinderivate substituierter Phenanthridine der Formel Ic werden erfindungsgemäß solche bevorzugt, worin R Wasserstoff bedeutet, die Amidinogruppen sich in 3- und 8-Stellung befinden, $R_1$ für Wasserstoff oder eine Alkylgruppe steht, $R_2$ und $R_3$ einzeln jeweils eine $C_1$-$C_6$-Alkylgruppe bedeuten oder zusammen mit dem Stickstoffatom, an das sie gebunden sind, eine heterozyklische Gruppe bilden ; $R_1$ und $R_2$ zusammen mit dem Kohlenstoffatom und dem Stickstoffatom, an das sie gebunden sind, einen Heterozyklus bilden, Z für Wasserstoff, Halogen, $C_1$-$C_6$-Alkoxy, Amino, $C_1$-$C_6$-Alkylamino, Di-$C_1$-$C_6$-alkylamino oder eine entsprechende heterozyklische Gruppe steht, sowie deren pharmazeutisch verträgliche Salze.

In der folgenden Tabelle sind einige neue Bisamidinderivate 6-substituierter Phenenthridine aufgeführt :

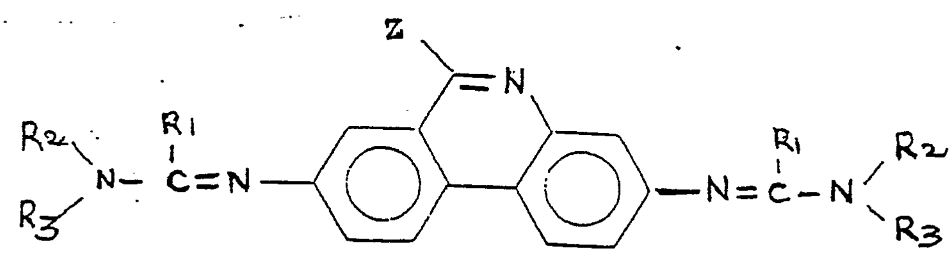

| $R_1$ | $R_2$ | $R_3$ | Z | Salz | Fp. (°C) |
|---|---|---|---|---|---|
| H | $CH_3$ | $CH_3$ | $N(C_2H_5)_2$ | - | 130 - 2 |
| H | $CH_3$ | $CH_3$ | $N(C_3H_7\text{-}n)_2$ | - | Öl |
| H | $C_2H_5$ | $C_2H_5$ | $N(CH_3)_2$ | - | 124 - 26 |
| H | $C_2H_5$ | $C_2H_5$ | $N(C_2H_5)_2$ | - | 126 - 7 |
| H | $C_2H_5$ | $C_2H_5$ | $N(C_3H_7\text{-}n)_2$ | - | Öl |

(Fortsetzung)

| $R_1$ | $R_2$ | $R_3$ | Z | Salz | Fp. (°C) |
|---|---|---|---|---|---|
| $CH_3$ | $CH_3$ | $CH_3$ | $N(CH_3)_2$ | - | 144 - 46 |
| $CH_3$ | $CH_3$ | $CH_3$ | $N(C_2H_5)_2$ | - | 130 - 2 |
| $CH_3$ | $CH_3$ | $CH_3$ | $N(C_3H_7\text{-}n)_2$ | - | 139 |
| $CH_3$ | $CH_3$ | $CH_3$ | $N(C_3H_7\text{-}n)_2$ | $3HCl \cdot 2H_2O$ | - |
| $CH_3$ | $C_2H_5$ | $C_2H_5$ | $N(CH_3)_2$ | - | 118 - 21 |
| $CH_3$ | $C_2H_5$ | $C_2H_5$ | $N(C_2H_5)_2$ | - | 132 - 33 |
| $CH_3$ | $C_2H_5$ | $C_2H_5$ | $N(C_2H_5)_2$ | $3HCl \cdot 3H_2O$ | 220 - 2 (Zers.) |
| $CH_3$ | $C_2H_5$ | $C_2H_5$ | $N(C_3H_7\text{-}n)_2$ | - | 146 - 47 |
| $CH_3$ | $C_2H_5$ | $C_2H_5$ | 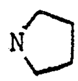 | - | 152 - 54 |
| $CH_3$ | $C_2H_5$ | $C_2H_5$ | 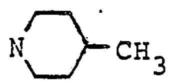 | - | 151 - 53 |
| $CH_3$ | $C_2H_5$ | $C_2H_5$ | 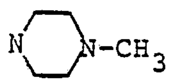 | - | 160 - 61 |
| $CH_3$ | $C_2H_5$ | $C_2H_5$ | 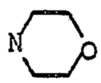 | - | 179 - 81 |
| $CH_3$ | $C_2H_5$ | $C_2H_5$ | 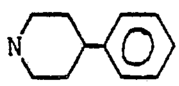 | - | 155 - 56 |
| $C_2H_5$ | $CH_3$ | $CH_3$ | $N(CH_3)_2$ | - | 160 - 62 |
| $C_2H_5$ | $CH_3$ | $CH_3$ | $N(C_2H_5)_2$ | - | 104 - 6 |
| $C_2H_5$ | $CH_3$ | $CH_3$ | $N(C_3H_7\text{-}n)_2$ | - | 111 - 13 |
| H | $-(CH_2)_4-$ | | $N(CH_3)_2$ | - | 202 - 3 |
| H | $-(CH_2)_4-$ | | $N(C_2H_5)_2$ | - | 149 - 50 |
| H | $-(CH_2)_4-$ | | $N(C_3H_7\text{-}n)_2$ | - | 183 - 86 |
| H | $-(CH_2)_5-$ | | $N(CH_3)_2$ | - | 164 - 65 |
| H | $-(CH_2)_5-$ | | $N(C_3H_7\text{-}n)_2$ | - | 134 - 36 |
| $-(CH_2)_3-$ | | H | $N(C_3H_7\text{-}n)_2$ | - | 167 - 70 |
| $-(CH_2)_3-$ | | $CH_3$ | $N(CH_3)_2$ | - | 181 - 82 |
| $-(CH_2)_3-$ | | $CH_3$ | $N(C_2H_5)_2$ | - | 144 - 45 |
| $-(CH_2)_3-$ | | $CH_3$ | $N(C_3H_7\text{-}n)_2$ | - | 152 - 53 |
| $-(CH_2)_3-$ | | $CH_3$ | 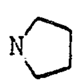 | - | 110 - 12 |
| $-(CH_2)_3-$ | | $CH_3$ | 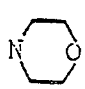 | - | 112 - 14 |
| $-(CH_2)_4-$ | | $CH_3$ | $N(C_2H_5)_2-$ | - | 135 - 37 |

(Fortsetzung)

| $R_1$ $R_2$ | $R_3$ | Z | Salz | Fp. (°C) |
|---|---|---|---|---|
| $-(CH_2)_4-$ | $CH_3$ | $N(C_3H_7\text{-}n)_2$ | - | 141 - 42 |
| $-(CH_2)_4-$ | $CH_3$ | 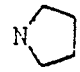 | - | 124 - 25 |
| $-(CH_2)_4-$ | $CH_3$ | 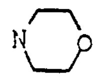 | - | 124 - 25 |

Gegenstand vorliegender Erfindung ist weiterhin ein Verfahren zur Herstellung der neuen Bisamidinderivate polyzyklischer Moleküle der Formel I.

Die Bisamidinderivate des 5,10-Dioxo-4,5,9,10-tetrahydro-4,9-dioxapyrens der Formel Ia bzw. deren pharmazeutisch verträgliche Salze werden hergestellt, indem man eine Verbindung der Formel II

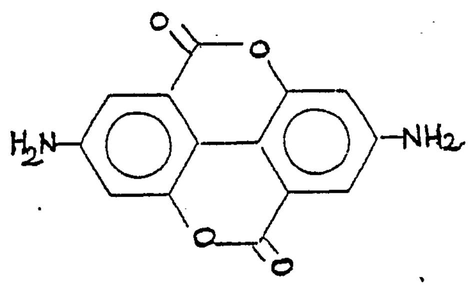

(II)

mit einem anorganischen Säurehalogenid wie z. B. Phosphoroxytrichlorid und einem Amid der Formel

$$R_1\overset{\overset{O}{\|}}{C}NR_2R_3$$

worin $R_1$, $R_2$ und $R_3$ die oben genannten Bedeutungen haben, reagieren läßt und gegebenenfalls die erhaltene Verbindung der Formel Ia auf bekannte Art und Weise in besagte Salze überführt.

Die Ausgangsverbindung der Formel II erhält man gemäß dem von Migachev., G.I. (Zh. Vses. Khim. Obstich. 24 (1979), 3, 307-309) beschriebenen Verfahren.

Die Reaktion der Verbindung der Formel II mit einem anorganischen Säurehalogenid und einem Amid der Formel

$$R_1\overset{\overset{O}{\|}}{C}NR_2R_3$$

kann durch Erhitzen des Reaktionsgemisches auf 50-80°C, vorzugsweise auf 60 °C, beschleunigt bzw. vervollständigt werden.

Die Reaktion kann gegebenenfalls in Lösungsmitteln wie halogenierten Kohenwasserstoffen, z. B. Chloroform, Methylenchlorid oder in Äthern wie Dioxan oder Tetrahydrofuran durchgeführt werden.

Bisamidinderivate des 6(5H)-Phenanthridinons der Formel Ib erhält man durch Reaktion des nach vom M. Devis (J. Chem. Soc (1956), 337) beschriebenen Verfahren erhältlichen 3,8-Diamino-6(5H)-phenanthridinons (III)

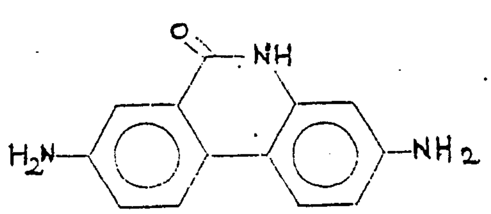

(III)

mit Phosphoroxychlorid und einem entsprechend substituierten Amid der Formel

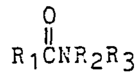

Durch Erhitzen des Reaktionsgemisches auf 50 °C kann die Reaktion beschleunigt bzw. vervollständigt werden. Gegebenenfalls kann die Reaktion in Lösungsmitteln wie halogenierten Kohlenwasserstoffen, beispielsweise Chloroform oder Methylenchlorid oder in Äthern, wie beispielsweise Dioxan oder Tetrahydrofuran durchgeführt werden.

Bisamidinderivate von Phenanthridinen der Formel Ic erhält man durch Reaktion einer Verbindung der Formel IV

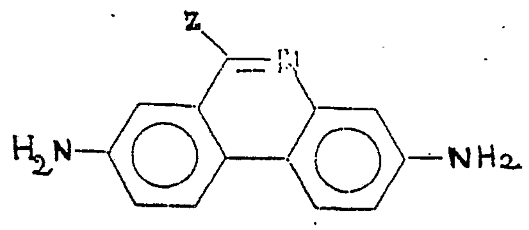

(IV)

worin R und Z die oben genannte Bedeutung besitzen, mit einem anorganischen Halogenid wie Phosphoroxytrichlorid und einem Amid der Formel

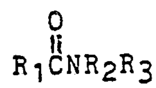

worin $R_1$, $R_2$ und $R_3$ die oben genannten Bedeutungen haben. Die entsprechenden pharmazeutisch verträglichen Salze erhält man durch Überführen der erhaltenen Verbindung der Formel Ic in besagte Salze auf übliche Weise.

Die Ausgangsverbindungen der Formel VI, worin R die oben genannte Bedeutung hat, Z für $C_1$-$C_6$-alkylamino, Di-$C_1$-$C_6$-alkylamino oder eine stickstoffhaltige heterozyklische Gruppe, die gegebenenfalls ein zweites Stickstoff-, Sauerstoff- oder Schwefelatom enthält, steht, erhält man durch Reaktion von 6-Chloro-3,8-dinitrophenanthridin (Albert A.I., Chem. Soc., 1284, 1948) mit entsprechenden Aminen auf bekannte Weise (Keene, S.R.T., Turner, G.L., Tetrahedron 27 (15), 3405, 971) und anschließender Reduktion der dabei erhaltenen Verbindung mit Hydrazinhydrat-Alkohol in Gegenwart eines Palladiumauf-Kohle Katalysators (Flechter, T.L., J. Med. Chem., 12, 822, 1969).

Die Reaktion der Verbindung der Formel IV mit Phosphoroxychlorid und einem Amid der Formel

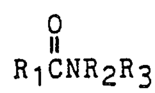

kann durch Erhitzen des Reaktionsgemisches auf 60-90 °C, vorzugsweise 80 °C, beschleunigt bzw. vervollständigt werden und kann gegebenenfalls auch in Lösungsmitteln wie halogenierten Kohlenwasserstoffen, beispielsweise Chloroform oder Methylenchlorid oder in Äthern wie Dioxan oder Tetrahydrofuran erfolgen.

Die Verbindung der Formel Ic kann man weiterhin herstellen durch Reaktion von Verbindungen der Formel Ib mit einem anorganischen Halogenid wie Phosphoroxychlorid, wobei man ein 6-Chlorderivat der Formel V erhält,

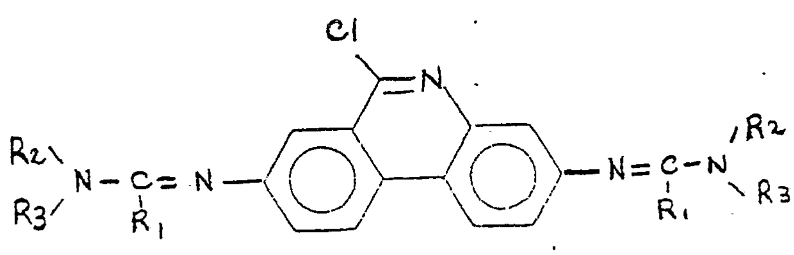

(V)

und anschließender Reaktion der erhaltenen Verbindung mit einem entsprechenden Amin.

Die erfindungsgemäßen substituierten Bisamidinderivate des 5,10-Dioxo-4,5,9,10-tetrahydro-4,9-dioxapyrens. des 6(5H)-Phenanthridinons und des 6-substituierten Phenanthridins zeichnen sich durch eine

antiprotozoische Wirksamkeit aus.

Die Verbindungen wurden auf ihre in vitro-Wirksamkeit in einem Monophasen-Medium auf einer polyaxenischem Entamoeba histolytica-Kultur getestet. Sie sind in einem Konzentrationsbereich von 50-200 mcg/ml wirksam.

Die in vivo Aktivität der Verbindungen wurden an Goldhamstern, die mit Hepatitisviren infiziert waren, nach der Methode von Jarumilinta (Ann. Trop. Med. Paras. 66, 2, 139-145, 1966) getestet. Die Verbindungen waren in Dosen von 4 × 30 mg/kg bis zu 4 × 200 mg/kg wirksam. Die in vivo Wirksamkeit gegen Blinddarm-Infektionen an Wistarratten wurde nach einer modifizierten Methode von Jones getestet (Ann. Trop. Med. Paras. 40, 130-140, 1946). Die Verbindungen erwiesen sich in Dosen von 4 × 225-300 mg/kg als wirksam.

Die in vitro- Prüfung auf die Wirksamkeit der Verbindungen gegen Trichomonaden wurden mit Trichomonas vaginalis auf modifiziertem CPLM Kulturboden durchgeführt (Chatterjee und Ray, J. Parasit., 65 (5) 815-816, 1979). Die Verbindungen erwiesen sich in Dosierungen von 100-200 mcg/ml als wirksam.

Die folgenden Beispiele erläutern die vorliegende Erfindung :

Beispiel 1

2,7-Di-(N',N'-dimethylaminomethylenamino)-5,10-dioxo-4,5-9,10-tetrahydro-4,9-dioxapyren-hydrochlorid

Ein Gemisch aus Phosphoroxychlorid (1,8 ml) in N,N-Diäthylformamid (4 ml), dessen Temperatur auf 5 °C gehalten wurde, wurde mit einer Aufschlämmung von 2,7-Diamino-5,10-dioxo-4,5,9,10-tetrahydro-4,9-dioxapyren (300 mg) in N,N-Diäthylformamid (6 ml) versetzt. Die erhaltene Mischung wurde 30 Minuten bei Zimmertemperatur gerührt, 5 Stunden bei 60 °C erhitzt, abgekühlt und mit Aceton verdünnt. Der entstandene Niederschlag wurde abfiltriert und in Wasser gelöst. Der pH-Wert der klaren Lösung wurde mit wässrigem Ammoniak auf 9 eingestellt. der sich bildende Niederschlag wurde abfiltriert, mit Wasser gewaschen, getrocknet und durch Säulenchromatographie einer Aluminiumoxidsäule und Benzol als Elutionsmittel gereinigt.

Der dabei erhaltene Feststoff wurde durch Reaktion mit ätherischer Salzsäurelösung in sein Hydrochlorid überführt. Das Hydrochlorid kristallisierte aus einem Äthanol-Äther-Gemisch aus. Ausbeute 55 %, Schm. 282 °C (Zers.).

Beispiel 2

2,7-Di-(N',N'-diäthylaminoäthylidenamino)5,10-dioxo-4,5,9,10-tetrahydro-4,9-dioxapyren

Wie in Beispiel 1, jedoch unter Verwendung von N,N-Diäthylacetamid anstelle von N,N-Diäthylformamid und ohne Überführung der erhaltenen Verbindung in ihr Hydrochlorid. Ausbeute 69 %, Schm. 220-222 °C ($CHCl_3$-Methanol).

Beispiel 3

2,7-Di-(N',N'-diäthylaminoäthylidenamino)-5,10-dioxo-4,5,9,10-tetrahydro-4,9-dioxapyren-hydrochlorid

Das in Beispiel 2 erhaltene 2,7-Di-(N',N'-diäthylaminoäthylidenamino)-5,10-dioxo-4,5,9,10-tetrahydrodioxapyren wurde gemäß dem in Beispiel 1, Absatz 2 beschriebenen Verfahren in sein Hydrochlorid überführt.

Ausbeute 88 %, Schm. 284-286 °C (Zers.) (Äthanol-Äther).

Beispiel 4

2,7-Di-(N-methylpyrrolidin-2-ylidenamino)-5,10-dioxo-4,5,9,10-tetrahydro-4,9-dioxapyren-hydrochlorid

Wie in Beispiel 1, jedoch unter Verwendung von N-Methyl-2-pyrrolidon anstelle von N,N-Diäthylformamid.

Ausbeute 58 %, Schm. 300 °C (Zers.) (Äthanol-Äther).

Beispiel 5

2,7-Di-(N-methylpiperid-2-ylidenamino)-5,10-dioxo-4,5,9,10-tetrahydro-4,9-dioxapyren-hydrochlorid

Wie Beispiel 1, jedoch unter Verwendung von N-Methyl-2-piperidon anstelle von N,N-Diäthylformamid.

Ausbeute 62 %, Schm. 277-279 °C (Zers.) (Äthanol-Äther).

Beispiel 6

2,7-Di-(piperidinomethylenamino)-5,10-dioxo-4,5,9,10-tetrahydro-4,9-dioxapyren-hydrochlorid-monohydrat

Wie Beispiel 1, jedoch unter Verwendung von N-Formyl-piperidin anstelle von N,N-Diäthylformamid. Ausbeute 59 %, Schm. 290-293 °C (Zers.) (Äthanol-Äther).

Beispiel 7

2,7-Di-(pyrrolidinomethylenamino)-5,10-dioxo-4,5,9,10-tetrahydro-4,9-dioxapyren

Ein Gemisch aus Phosphoroxychlorid (0,7 ml) in N-Formylpyrrolidin (2 ml) und Tetrahydrofuran (4 ml), das bei einer Temperatur von 5 °C gehalten wurde, wurde mit einer Aufschlämmung aus 2,7-Diamino-5,10-dioxo-4,5,9,10-tetrahydro-4,9-dioxapyren (500 mg) in Tetrahydrofuran (4 ml) versetzt. Die erhaltene Mischung wurde 30 Minuten bei Zimmertemperatur gerührt, 1 Stunde bei 80 °C erhitzt und wie in Beispiel 1, Absatz 1 beschrieben, aufgearbeitet.

Ausbeute 68 %, Schm. 275 °C (Zers.) ($CHCl_3$-Methanol).

Beispiel 8

3,8-Di-(N',N'-dimethylaminoäthylidenamino)-6(5H)-phenanthridinon-dihydrochlorid-dihydrat

Ein Gemisch aus Phosphoroxychlorid (1,5 ml) in N,N-Dimethylacetamid (5 ml), das bei einer Temperatur von 5 °C gehalten wurde, wurde mit einer Aufschlämmung aus 3,8-Diamino-6(5H)-phenanthridinon (500 mg) in N,N-Dimethylacetamid (1,5 ml) versetzt. Die erhaltene Mischung wurde 30 Minuten bei Zimmertemperatur gerührt, 1 Stunde bei 45-50 °C erhitzt, abgekühlt und mit Aceton verdünnt. Der dabei erhaltene Niederschlag wurde abfiltriert und in Wasser gelöst. Der pH-Wert der klaren Lösung wurde mit wässrigem Ammoniak auf 9-10 eingestellt. Der dabei entstehende Feststoff wurde filtriert, mit Wasser gewaschen, getrocknet und säulenchromatographisch mit einer Aluminiumoxidkolonne unter Verwendung von Benzol und Chloroform als Laufmittel gereinigt. Der anfallende Feststoff wurde in sein Hydrochlorid überführt und aus Methanol-Äther umkristallisiert.

Ausbeute 85 %, Schm. 268-270 °C (Zers.).

Beispiel 9

3,8-Di-(N',N'-dimethylaminomethylenamino)-6(5H)-phenanthridinon

Wie Beispiel 8, jedoch unter Verwendung von N,N-Diäthylformamid anstelle von N,N-Dimethylacetamid und ohne Überführung der erhaltenen Verbindung in das Hydrochlorid. Das Produkt kristallisierte aus Chloroform-Methanol aus.

Ausbeute 75 %, Schm. 240-241 °C (Zers.).

Beispiel 10

Die in Beispiel 9 erhaltene Verbindung wurde in ihr Hydrochlorid gemäß dem in Beispiel 1, Absatz 2 beschriebenen Verfahren überführt.

Ausbeute 90 %, Schm. 281-283 °C (Zers.) (Äthanol-Äther).

Beispiel 11

3,8-Di-(N-methylpyrrolidin-2-ylidenamino)-6(5H)-phenanthridinon-dihydrochlorid-dihydrat

Wie Beispiel 8, jedoch unter Verwendung von N-Methyl-2-pyrrolidon anstelle von N,N-Dimethylacetamid.

Ausbeute 72 %, Schm. 255 °C (Zers.).

Beispiel 12

3,8-Di-(piperidinomethylenamino)-6(5H)-phenanthridinondihydrochlorid

Wie Beispiel 8, jedoch unter Verwendung von N-Formylpiperidin anstelle von N,N-Dimethylacetamid. Ausbeute 86 %, Schm. 255-257 °C (Zers.).

Beispiel 13

3,8-Di-(pyrrolidinomethylenamino)-6(5H)-phenanthridinon

Ein Gemisch aus Phosphoroxytrichlorid (0,75 ml) in N-Formylpyrrolidin (2,5 ml) und Tetrahydrofuran (5 ml), das bei einer Temperatur von 5 °C gehalten wurde, wurde mit einer Aufschlämmung von 3,8-Diamino-6(5H)-phenanthridinon (500 mg) in Tetrahydrofuran (5 ml) versetzt. Die erhaltene Mischung wurde 30 Minuten bei Zimmertemperatur gerührt, 1 Stunde bei 50 °C erhitzt und wie in Beispiel 8, Absatz 1 beschrieben, aufgearbeitet.

Ausbeute 52 %, Schm. 280 °C (Zers.) ($CHCl_3$-Methanol).

Beispiel 14

3,8-Di-(N-diäthylaminomethylenamino)-6-dimethylaminophenanthridin

Ein Gemisch aus Phosphoroxytrichlorid (1,5 ml) in N,N-Diäthylformamid (5 ml), das bei einer Temperatur von 5 °C gehalten wurde, wurde mit einer Aufschlämmung aus 3,8-Diamino-6-dimethylaminophenanthridin (500 mg) in N,N-Diäthylformamid (2 ml) versetzt. Die erhaltene Mischung wurde 30 Minuten bei Zimmertemperatur gerührt, 2 Stunden bei 75-80 °C erhitzt, abgekühlt und mit Aceton verdünnt. Der entstandene Niederschlag wurde abfiltriert und in Wasser gelöst. Der pH-Wert der klaren Lösung wurde mit wässrigem Ammoniak auf 9 eingestellt. Der dabei anfallende Feststoff wurde abfiltriert, mit Wasser gewaschen, getrocknet und säulenchromatographisch mittels einer neutralen Aluminiumoxidsäule unter Verwendung von Benzol als Laufmittel gereinigt. Das Produkt kristallisierte aus Chloroform-Di-isopropyläther aus.

Ausbeute 71 %, Schm. 124-126 °C.

Beispiel 15

6-Diäthylamino-3,8-di-(pyrrolidinomethylenamino)-phenanthridin

Wie Beispiel 14, jedoch unter Verwendung von 3,8 Diamino-6-diäthylaminophenanthridin und N-Formylpyrrolidin anstelle von 3,8-Diamino-6-dimethylaminophenanthridin und N,N-Diäthylformamid.

Ausbeute 66 %, Schm. 149-150 °C (Chloroform-Petroleumäther (60-80 °C)).

Beispiel 16

3,8-Di-(N,N-dimethylaminoäthylidenamino)-6-dipropylaminophenanthridin

Wie Beispiel 14, jedoch unter Verwendung von 3,8-Diamino-6-dipropylaminophenanthridin und N,N-Dimethylacetamid anstelle von 3,8-Diamino-6-dimethylaminophenanthridin und N,N-Diäthylformamid.

Ausbeute 60 %, Schm. 139 °C (Chloroform-Diisopropyläther).

Beispiel 17

3,8-Di-(N,N-dimethylaminoäthylidenamino)-6-dipropylaminophenanthridin-trihydrochlorid

Das in Beispiel 16 erhaltene 3,8-Di-(N,N-dimethylaminoäthylidenamino-6-dipropylaminophenanthridin wurde durch Behandlung mit ätherischer Salzsäurelösung in Methanol in sein Hydrochlorid überführt.

Ausbeute 95 %.

Beispiel 18

3,8-Di-(N-methylpyrrolidin-2-ylidenamino)-6-morpholinophenanthridin

Wie Beispiel 14, jedoch unter Verwendung von 3,8-Diamino-6-morpholinophenanthridin und N-Methyl-2-pyrrolidon anstelle von 3,8-Diamino-6-dimethylaminophenanthridin und N,N-Diäthylformamid.

Ausbeute 35 %, Schm. 112-114 °C (Petroleumäther (60-80 °C)).

Beispiel 19

3,8-Di-(N,N-diäthylaminoäthylidenamino)-6-(N'-methylpiperazino)-phenanthridin

Wie Beispiel 14, jedoch unter Verwendung von 3,8-Diamino-6-(N-methylpiperazino)-phenanthridin und N,N-Diäthylacetamid anstelle von 3,8-Diamino-6-dimethylaminophenanthridin und N,N-Diäthylformamid.

Ausbeute 78 %, Schm. 160-161 °C (Diisopropyläther-Petroleumäther (60-80 °C)).

Beispiel 20

3,8-Di-(N,N-diäthylaminoäthylidenamino)-6-(4-phenylpiperidino)-phenanthridin

Wie Beispiel 14, jedoch unter Verwendung von 3,8-Diamino-6-(4-phenylpiperidino)-phenanthridin und N,N-Diäthylacetamid anstelle von 3,8-Diamino-6-dimethylaminophenanthridin und N,N-Diäthylformamid.

Ausbeute 75 %, Schm. 155-156 °C (Diisopropyläther).

Beispiel 21

3,8-Di-(N-methylpyrrolidin-2-ylidenamino)-6-dipropylaminophenanthridin

Ein Gemisch aus Phosphoroxytrichlorid (1,5 ml) in N-Methyl-2-pyrrolidon (5 ml) und Tetrahydrofuran (4 ml), das bei einer Temperatur von 5 °C gehalten wurde, wurde mit einer Aufschlämmung aus 3,8-Diamino-6-dipropylaminophenanthridin (500 mg) in Tetrahydrofuran (4 ml) versetzt. Die erhaltene Mischung wurde 30 Minuten bei Zimmertemperatur gerührt, 3 Stunden bei 80 °C erhitzt und wie in Beispiel 14 beschrieben, aufgearbeitet.

Ausbeute 62 %, Schm. 152-152 °C (Diisopropyläther-Petroleumäther (60-80 °C)).

Beispiel 22

3,8-Di-(N-methylpiperidin-2-ylidenamino)-6-pyrrolidinophenanthridin

Wie Beispiel 21, jedoch unter Verwendung von 3,8-Diamino-6-pyrrolidinophenanthridin anstelle von 3,8-Diamino-6-dipropylaminophenanthridin und unter Verwendung von Dioxan anstelle von Tetrahydrofuran.

Ausbeute 28 %, Schm. 124-125 °C (Chloroform-Petroleumäther (60-80 °C)).

**Patentansprüche**

1. Verbindungen der allgemeinen Formel I

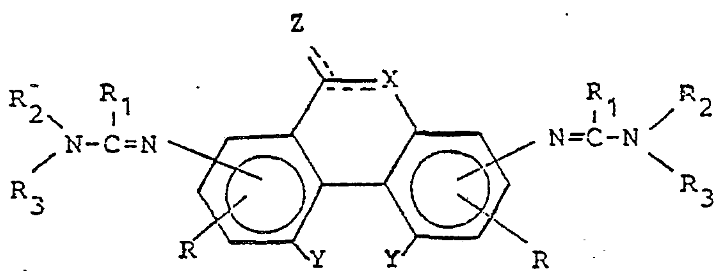

(I)

worin

R Wasserstoff, $C_1$-$C_3$-Alkyl, $C_1$-$C_3$-Alkoxy, Halogen, Nitro oder Amino,

$R_1$ Wasserstoff, eine nicht substituierte oder mit der Amino- oder Di-$C_1$-$C_3$-alkylaminogruppe substituierte $C_1$-$C_6$-Alkylgruppe, oder eine Di-$C_1$-$C_3$-alkyl-aminogruppe,

$R_2$ und $R_3$ einzeln eine $C_1$-$C_6$-Alkylgruppe oder, zusammen mit dem Stickstoffatom, an das sie gebunden sind, eine heterozyklische Gruppe bedeuten, oder

$R_1$ und $R_2$ zusammen mit dem Kohlenstoffatom und dem Stickstoffatom, an das sie gebunden sind, eine heterozyklische Gruppe bedeuten,

X und Z jeweils Sauerstoff bedeuten oder

X für die —NH-Gruppe und Z für Sauerstoff steht oder

X für Stickstoff und Z für Wasserstoff, Halogen, $C_1$-$C_6$-Alkoxy, Amino, Mono-$C_1$-$C_6$-alkylamino, Di-$C_1$-$C_6$-alkylamino oder eine heterozyklische Gruppe,

Y für Wasserstoff oder, falls X und Z jeweils Sauerstoff bedeuten, für die Brücke

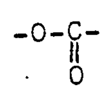

die ein zusätzliches Ringsystem bildet,

steht und deren pharmazeutisch verträgliche Salze.

2. Verbindungen gemäß Anspruch 1, mit der allgemeinen Formel Ia

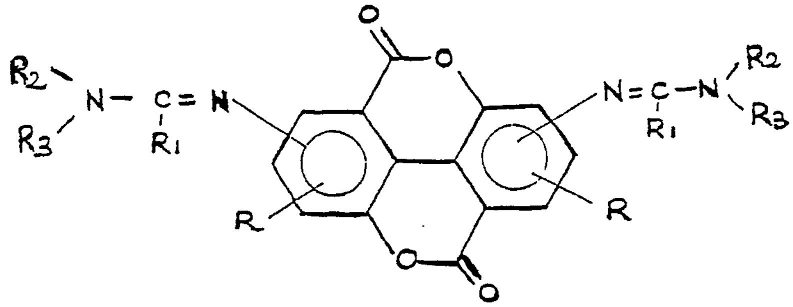

(Ia)

worin R, $R_1$, $R_2$ und $R_3$ die in Anspruch 1 angegebenen Bedeutungen besitzen.

3. Verbindungen gemäß Anspruch 1 mit der allgemeinen Formel Ib

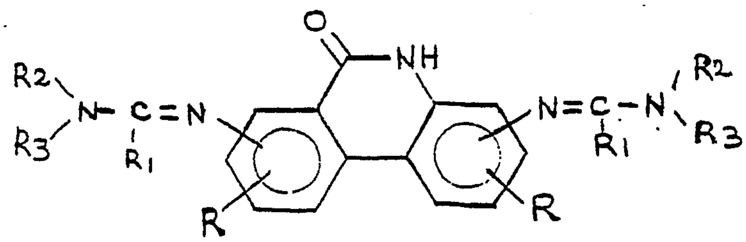

(Ib)

worin R, $R_1$, $R_2$ und $R_3$ die in Anspruch 1 angegebenen Bedeutungen besitzen.

4. Verbindungen gemäß Anspruch 1 mit der allgemeinen Formel Ic

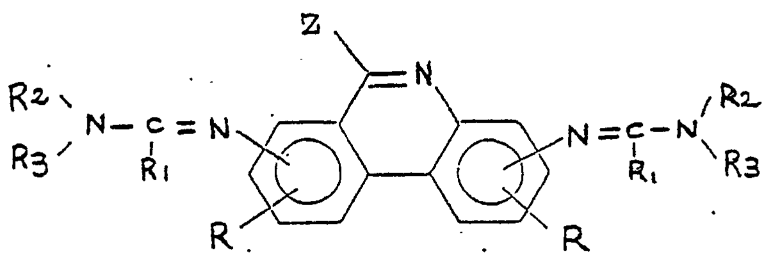

(Ic)

worin R, $R_1$, $R_2$ und $R_3$ die in Anspruch 1 angegebenen Bedeutungen besitzen und Z Wasserstoff, Halogen, eine $C_1$-$C_6$-Alkoxy-, Amino-, $C_1$-$C_6$-Alkylamino-, Di-$C_1$-$C_6$-Alkylamino- oder eine heterozyklische Gruppe bedeuten.

5. Verbindungen gemäß Anspruch 4 der allgemeinen Formel Ic worin Z in der Bedeutung einer heterozyklischen Gruppe für gegebenenfalls durch eine oder mehrere $C_1$-$C_3$-Alkylgruppen substituiertes Pyrrolidin, Piperidin, Imidazolin oder Pyrimidin steht.

6. Verfahren zur Herstellung von Verbindungen gemäß Anspruch 1, dadurch gekennzeichnet, daß man eine Verbindung der Formel V

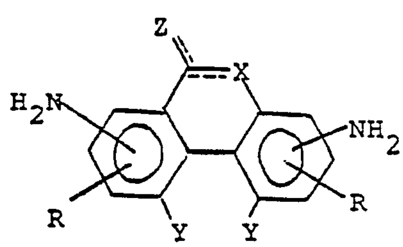

(V)

worin

R Wasserstoff, $C_1$-$C_3$-Alkyl, $C_1$-$C_3$-Alkoxy, Halogen, Nitro oder Amino,

X und Z entweder gleichzeitig Sauerstoff, oder

X die —NH-Gruppe und Z Sauerstoff oder

X Stickstoff und Z Wasserstoff, Halogen, $C_1$-$C_6$-Alkoxy, Amino, $C_1$-$C_6$-Alkylamino, Die-$C_1$-$C_6$-alkylamino oder eine heterozyklische Gruppe,

Y Wasserstoff oder, falls X und Z jeweils Sauerstoff bedeuten, die Brücke

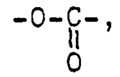

die ein zusätzliches Ringsystem bildet, bedeuten, mit einem anorganischen Säurehalogenid und einem Amid der Formel

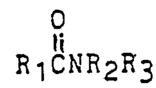

worin

$R_1$ Wasserstoff, eine unsubstituierte oder mit Amino oder Di-$C_1$-$C_3$-alkylamino substituierte $C_1$-$C_6$-Alkylgruppe, oder eine Di-$C_1$-$C_3$-alkylaminogruppe,

$R_2$ und $R_3$ einzeln jeweils eine $C_1$-$C_6$-Alkylgruppe oder zusammen mit dem Stickstoffatom, an das sie gebunden sind, eine heterozyklische Gruppe bedeuten, oder

$R_1$ und $R_2$ zusammen mit dem Kohlenstoffatome und dem Stickstoffatom, an welche sie gebunden sind, eine heterozyklische Gruppe bedeuten,

umsetzt.

7. Verfahren nach Anspruch 6, dadurch gekennzeichnet, daß die Umsetzung bei einer Temperatur von 50-90 °C erfolgt.

8. Verfahren zur Herstellung einer Verbindung der Formel I, worin R, $R_1$ und $R_2$ die zur Formel I in Anspruch 6 genannte Bedeutung haben, Y Wasserstoff, X ein Stickstoffatom und Z Amino, $C_1$-$C_6$-Alkylamino, Di-$C_1$-$C_6$-alkylamino oder einen heterozyklischen Rest darstellen, dadurch gekennzeichnet, daß man eine Verbindung der Formel Ib

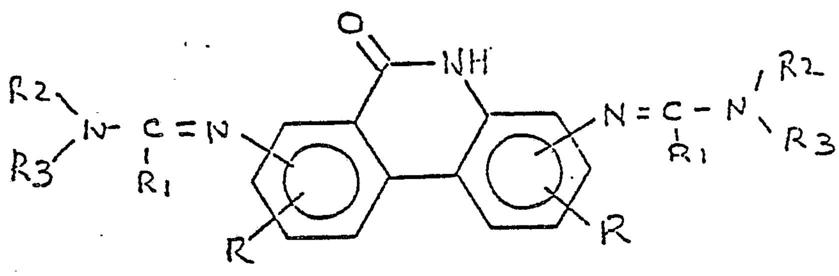

(Ib)

mit einem anorganischen Halogenierungsmittel umsetzt zu einer Verbindung der Formel

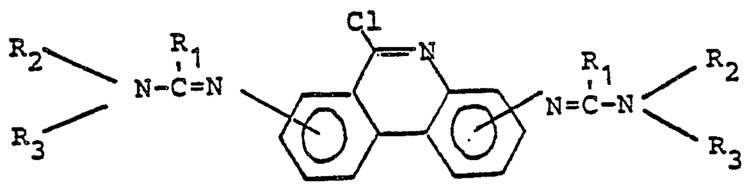

und die erhaltene Verbindung mit einem entsprechenden Amin oder einem Heterozyklus umsetzt.

9. Pharmazeutisches Präparat, gekennzeichnet durch eine wirksame Menge einer Verbindung der Formel I gemäß Anspruch 1 als aktiven Wirkstoff in Mischung oder Verbindung mit einer pharmazeutisch verträglichen Träger und/oder Stabilisator.

10. Verwendung einer Verbindung der Formel I gemäß Anspruch 1 zur Herstellung von Arzneimitteln mit Wirkung gegen Protozoen und Trichomonaden.

**Claims**

1. A compound of the formula I

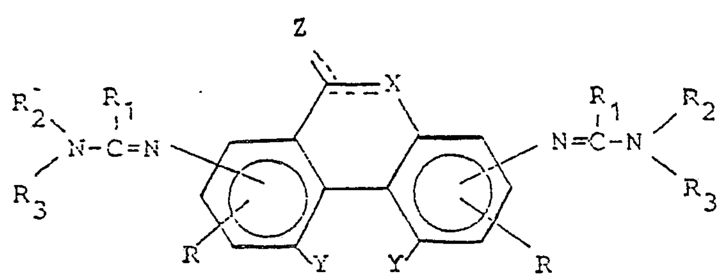

(I)

in which

R denotes hydrogen, $C_1$-$C_3$-alkyl, $C_1$-$C_3$-alkoxy, halogen, nitro or amino,

$R_1$ denotes hydrogen, a $C_1$-$C_6$-alkyl group which is unsubstituted or substituted by the amino or di-$C_1$-$C_3$-alkylamino group, or a di-$C_1$-$C_3$-alkylamino group,

$R_2$ and $R_3$ individually denote a $C_1$-$C_6$-alkyl group or, together with the nitrogen atom to which they are attached, denote a heterocyclic group, or

$R_1$ and $R_2$, together with the carbon atom and the nitrogen atom to which they are attached, denote a heterocyclic group,

X and Z each denote oxygen or

X represents the —NH- group and Z represents oxygen or

X represents nitrogen and Z represents hydrogen, halogen, $C_1$-$C_6$-alkoxy, amino, mono-$C_1$-$C_6$-alkylamino di-$C_1$-$C_6$-alkylamino or a heterocyclic group, and

Y represents hydrogen or, if X and Z each denote oxygen, represents the bridge

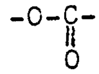 ,

which forms an additional ring system,
and pharmaceutically acceptable salts thereof.

2. A compound as claimed in claim 1, of the formula Ia

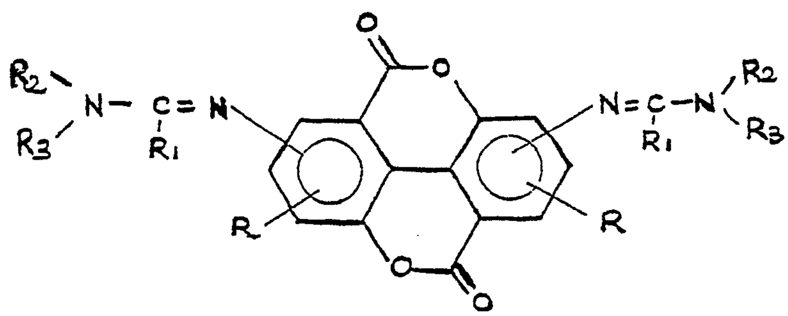

(Ia)

in which R, $R_1$, $R_2$ and $R_3$ have the meanings indicated in claim 1.

3. A compound as claimed in claim 1 of the formula Ib

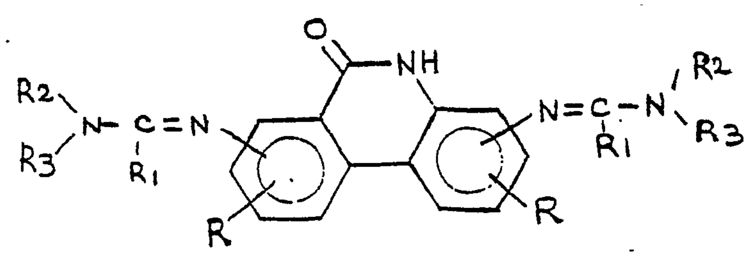

(Ib)

in which R, $R_1$, $R_2$ and $R_3$ have the meanings indicated in claim 1.

4. A compound as claimed in claim 1 of the formula Ic

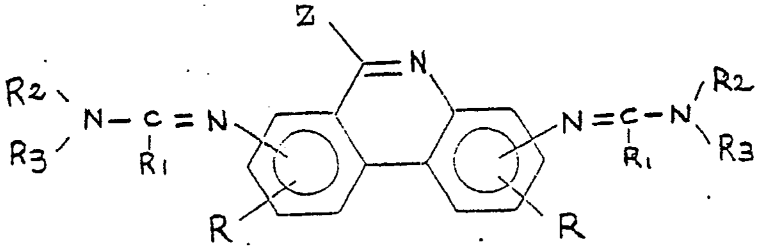

(Ic)

in which R, $R_1$, $R_2$ and $R_3$ have the meanings indicated in claim 1, and Z denotes hydrogen, halogen, a $C_1$-$C_6$-alkoxy, amino, $C_1$-$C_6$-alkylamino or di-$C_1$-$C_6$-alkylamino group or a heterocyclic group.

5. A compound as claimed in claim 4 of the formula Ic in which Z, when it denotes a heterocyclic group, represents pyrrolidine, piperidine, imidazoline or pyrimidine which is optionally substituted by one

or more $C_1$-$C_3$-alkyl groups.

6. A process for the preparation of a compounds as defined in claim 1 which comprises reacting a compound of the formula V

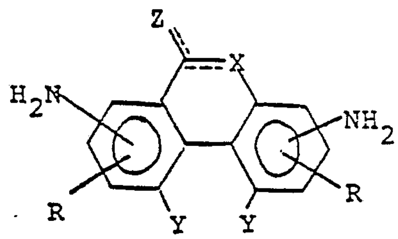

(V)

in which

R denotes hydrogen, $C_1$-$C_3$-alkyl, $C_1$-$C_3$-alkoxy, halogen, nitro or amino,

X and Z either simultaneously denote oxygen, or

X denotes the —NH- group and Z denotes oxygen, or

X denotes nitrogen and Z denotes hydrogen, halogen, $C_1$-$C_6$-alkoxy, amino, $C_1$-$C_6$-alkylamino, di-$C_1$-$C_6$-alkylamino or a heterocyclic group, and

Y denotes hydrogen or, if X and Z each denote oxygen, the bridge

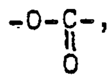

which forms an additional ring system,
with an inorganic acid halide and an amide of the formula

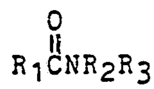

in which

$R_1$ denotes hydrogen, a $C_1$-$C_6$-alkyl group which is unsubstituted or substituted by amino or di-$C_1$-$C_3$-alkylamino, or a di-$C_1$-$C_3$-alkylamino group,

$R_2$ and $R_3$ each individually denote a $C_1$-$C_6$-alkyl group or, together, with the nitrogen atom to which they are attached, denote a heterocyclic group, or

$R_1$ and $R_2$, together with the carbon atom and the nitrogen atom to which they are attached, denote a heterocyclic group.

7. The process as claimed in claim 6, wherein the reaction is carried out at a temperature of 50-90 °C.

8. A process for the preparation of a compound of the formula I in which R, $R_1$ and $R_2$ have the meaning mentioned for formula I in claim 6, Y represents hydrogen, X represents a nitrogen atom and Z represents amino, $C_1$-$C_6$-alkylamino, di-$C_1$-$C_6$-alkylamino or a heterocyclic radical, which comprises reacting a compound of the formula Ib

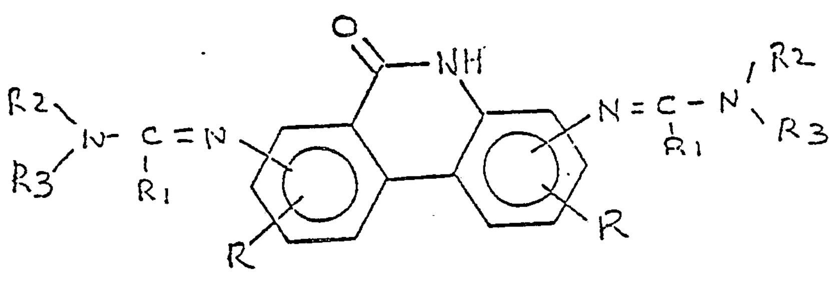

(Ib)

with an inorganic halogenating agent to give a compound of the formula

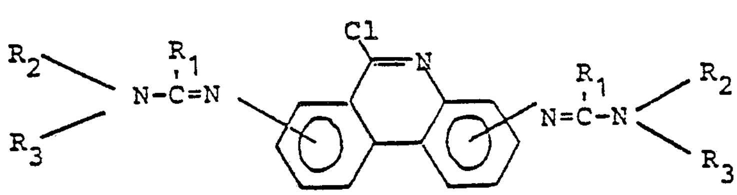

and reacting the resulting compound with an appropriate amine or a heterocyclic compound.

9. A pharmaceutical product which contains an effective amont of a compound of the formula I as claimed in claim 1 as the active compound, in a mixture or combination with a pharmaceutically acceptable excipient and/or stabilizer.

10. The use of a compound of the formula I as claimed in claim 1 for the preparation of medicaments having activity against protozoal infections.

**Revendications**

1. Composés de formule générale I

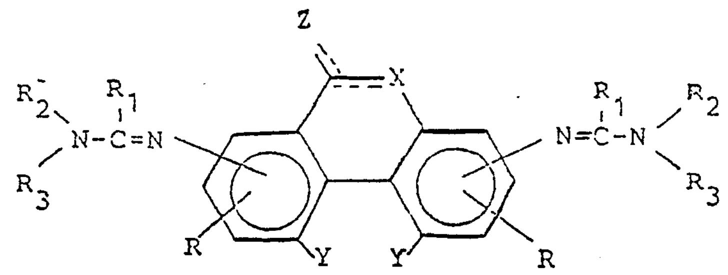

(I)

dans laquelle

R représente l'hydrogène, un groupe alcoyle en $C_1$-$C_3$, alcoxy en $C_1$-$C_3$, un halogène, un groupe nitro ou amino,

$R_1$ représente l'hydrogène, un groupe alcoyle en $C_1$-$C_6$ non-substitué ou substitué avec un groupe amino ou di-alcoyl ($C_1$-$C_3$)-amino, ou un groupe di-alcoyl ($C_1$-$C_3$) amino,

$R_2$ et $R_3$ représentent chacun un groupe alcoyle en $C_1$-$C_6$, ou forment ensemble un groupe hétérocyclique avec l'atome d'azote sur lequel ils sont fixés, ou

$R_1$ et $R_2$ forment ensemble un groupe hétérocyclique, avec l'atome de carbone et l'atome d'azote sur lequel ils sont fixés,

X et Z représentent chacun l'oxygène ou

X représente le groupe —NH— et Z représente l'oxygène ou

X représente l'azote et Z représente l'hydrogène, un halogène, un groupe alcoxy en $C_1$-$C_6$, amino, monoalcoyl ($C_1$-$C_6$)-amino, dialcoyl($C_1$-$C_6$)-amino ou un groupe hétérocyclique,

Y représente l'hydrogène ou, dans le cas où X et Z représentent chacun l'oxygène, le pont

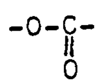 ,

qui forme un système cyclique supplémentaire ;
et leurs sels pharmaceutiquement acceptables.

2. Composés selon la revendication 1, de formule générale Ia

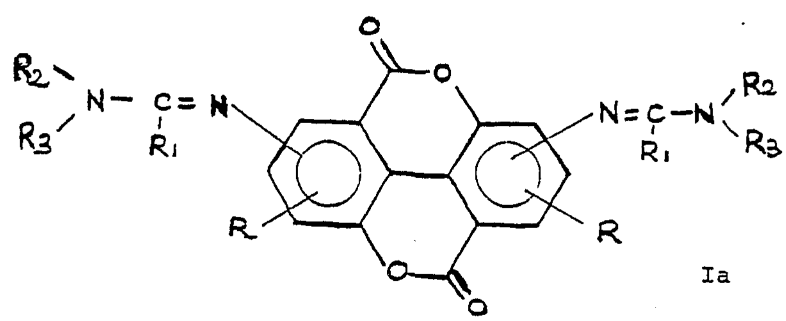

(Ia)

Ia

dans laquelle R, $R_1$, $R_2$ et $R_3$ ont les significations indiquées dans la revendication 1.

3. Composés selon la revendication 1, de formule générale Ib

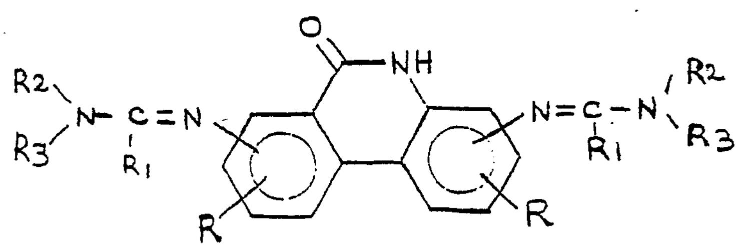

(Ib).

dans laquelle R, $R_1$, $R_2$ et $R_3$ ont les significations indiquées dans la revendication 1.

4. Composés selon la revendication 1, de formule générale Ic

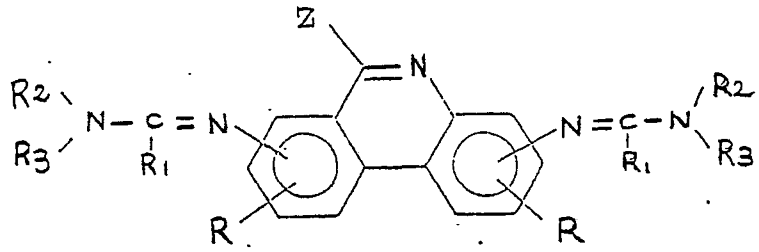

(Ic)

dans laquelle R, $R_1$, $R_2$ et $R_3$ ont les significations indiquées dans la revendication 1 et Z représente l'hydrogène, un halogène, un groupe alcoxy en $C_1$-$C_6$, amino, alcoyl($C_1$-$C_6$)-amino, di-alcoyl-($C_1$-$C_6$)-amino ou un groupe hétérocyclique.

5. Composés selon la revendication 4, de formule générale Ic, dans laquelle Z étant un groupe hétérocyclique représente la pyrrolidine, la pipéridine, l'imidazoline ou la pyrimidine, éventuellement substituée par un ou plusieurs groupes alcoyle en $C_1$-$C_3$.

6. Procédé de préparation de composés suivant la revendication 1 caractérisé en ce qu'on fait réagir un composé de formule V

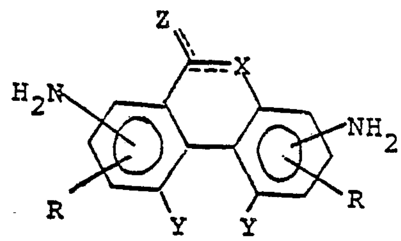

(V)

dans laquelle

R représente l'hydrogène, un groupe alcoyle en $C_1$-$C_3$, alcoxy en $C_1$-$C_3$, un halogène, un groupe nitro ou amino,

X et Z représentent simultanément l'oxygène ou

X représente le groupe —NH— et Z représente l'oxygène ou

X représente l'azote et Z représente l'hydrogène, un halogène, un groupe alcoxy en $C_1$-$C_6$, amino, alcoyl ($C_1$-$C_6$)-amino, di-alcoyl($C_1$-$C_6$)-amino ou un groupe hétérocyclique,

Y dans le cas où X et Z représentent chacun l'oxygène, représente le pont

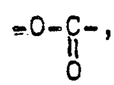

qui forme un système cyclique supplémentaire.
avec un halogénure d'acide minéral ou un amide de formule

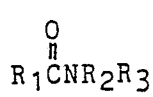

dans laquelle

$R_1$ représente l'hydrogène, un groupe alcoyle en $C_1$-$C_6$ non-substitué ou substitué par un groupe amino ou di-alcoyl ($C_1$-$C_3$)-amino, ou un groupe di-alcoyl ($C_1$-$C_3$)-amino,

$R_2$ et $R_3$ représentent chacun un groupe alcoyle en $C_1$-$C_6$, ou forment ensemble un groupe hétérocyclique avec l'atome d'azote sur lequel ils sont fixés, ou

$R_1$ et $R_2$ forment ensemble un groupe hétérocyclique avec l'atome de carbone et l'atome d'azote sur lesquels ils sont fixés.

7. Procédé selon la revendication 6, caractérisé en ce que la réaction est effectuée à une température comprise entre 50 et 90 °C.

8. Procédé de préparation d'un composé de formule I, dans laquelle R, $R_1$ et $R_2$ ont les significations indiquées pour la formule I dans la revendication 6, Y représente l'hydrogène, X représente un atome d'azote et Z représente un groupe amino, alcoyl($C_1$-$C_6$)-amino, di-alcoyl($C_1$-$C_6$)-amino ou un groupe hétérocyclique, caractérisé en ce qu'on fait réagir un composé de formule Ib

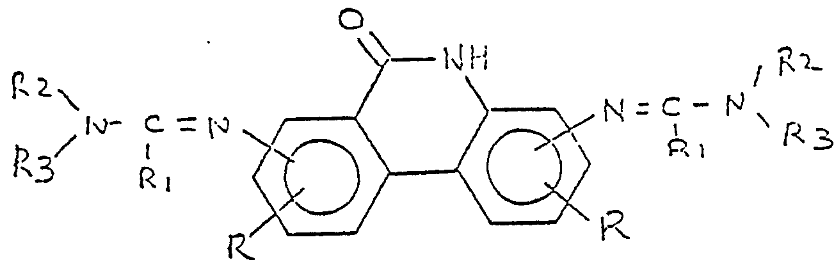

(Ib)

avec un agent d'halogénation inorganique pour obtenir un composé de formule

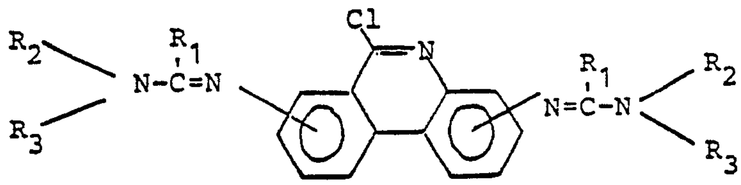

puis on fait réagir le composé obtenu avec une amine correspondante ou un hétérocycle.

9. Composition pharmaceutique, caractérisée en ce qu'elle contient une quantité efficace d'un composé de formule I selon la revendication 1 comme substance active, en mélange ou en combinaison avec un véhicule et/ou un stabilisant pharmaceutiquement acceptable.

10. Utilisation d'un composé de formule I selon la revendication 1 pour la préparation de médicaments agissant contre les protozoaires et les trichomonades.